# EUROPEAN PATENT SPECIFICATION

(11) **EP 2 849 817 B1**
(45) Date of publication and mention of the grant of the patent: **13.03.2019**
(21) Application number: 13791497.4
(22) Date of filing: 14.03.2013
(51) Int. Cl.: A61M 1/36, A61M 1/38, A61M 39/08

(54) **SYSTEMS FOR EXTRACORPOREAL BLOOD MODIFICATION**
SYSTEME ZUR EXTRAKORPORALEN BLUTMODIFIKATION
SYSTÈMES POUR LA MODIFICATION EXTRACORPORELLE DU SANG

(30) Priority: 14.05.2012 US 201261646674 P
(43) Date of publication of application: 25.03.2015
(73) Proprietor: Children's Medical Center Corporation, Boston, MA 02115 (US)
(72) Inventor: MCALVIN, James, B., West Roxbury, MA 02132 (US); MIZRAHI, Boaz, Brookline, MA 02446 (US); KOHANE, Daniel, S., Newton, MA 02461 (US); WYLIE, Ryan, G., Boston, MA 02215 (US)
(74) Representative: Potter Clarkson
(86) International application number: PCT/US2013/031744
(87) International publication number: WO 2013/172966

(56) References cited:
- WO-A2-2011/044329
- WO-A2-2011/112873
- US-A1- 2003 120 202
- US-A1- 2005 242 033
- US-A1- 2009 191 175
- US-A1- 2011 098 623
- US-A1- 2011 098 623
- ZHI-YONG PENG ET AL: "Acute removal of common sepsis mediators does not explain the effects of extracorporeal blood purification in experimental sepsis", KIDNEY INTERNATIONAL, vol. 81, no. 4, 1 February 2012 (2012-02-01), pages 363-369, XP055236195, LONDON, GB ISSN: 0085-2538, DOI: 10.1038/ki.2011.320

## Description

### RELATED APPLICATIONS

This application claims the benefit of U.S. Provisional Patent Application Serial No. 61/646,674, filed May 14, 2012, entitled "Systems and Methods for Extracorporeal Blood Modification," by McAlvin, *et al.*

### FIELD OF THE DISCLOSURE

The present disclosure generally relates to devices, systems, and methods for modification of biological fluids, e.g., targeted removal of proteins or other biomolecules from the blood of a subject.

### BACKGROUND

Sepsis, severe sepsis, and septic shock affect millions of people worldwide each year and are the leading causes of death in critically ill patients in the United States. The prevailing theory has been that sepsis represents an uncontrolled inflammatory response incited by infection. Animal models result in "cytokine storms" upon induction of septic shock, whether by injection of bacteria, lipopolysaccharide administration, or cecal ligation and puncture. Inflammatory cytokines such as TNF-alpha (TNF-α), IL-1beta (IL-1β), IL-6, VEGF, and many more have been implicated as the culprits for the sepsis syndrome and its associated features. Attempts to modulate the inflammatory cascade have been largely unsuccessful, including trials of corticosteroids, antiendotoxin antibodies, TNF-alpha (TNF-α) antagonists, IL-1 receptor antagonists, ibuprofen, and extracorporeal blood modification (e.g., plasmapheresis). One reason for their failure may be because the sepsis syndrome changes over time. The early phase is characterized by a surge of inflammatory cytokines. But as sepsis persists, a shift toward an immunosuppressive state develops and is mediated by anti-inflammatory cytokines such as IL-10 and TGF-beta (TGF-β). Anti-inflammatory therapies that may be beneficial during the initial phase may worsen the immune suppression that ensues due to their long-acting effects. Although the effects of extracorporeal immune modulation (e.g., hemofiltration, plasma exchange, plasmapheresis) are limited to the time that they are in use, they lack selectivity and may remove anti-inflammatory cytokines. Accordingly, improvements in treatment are needed.

### SUMMARY OF INVENTION

In the following description, various optional exemplary embodiments and aspects are disclosed that do not form part of the invention but are described merely to aid in understanding the invention. The present invention is defined in the accompanying claims. Embodiments of the invention are described in the following numbered paragraphs:
(1). A device, comprising:
   an extracorporeal circuit comprising a tube having an inner surface and an antibody or antigen-binding fragment thereof bound to a substrate forming the inner surface of the tube, wherein the substrate comprises a polymeric microgel.
(2). The device of paragraph 1, wherein the antibody or antigen-binding fragment thereof is specific to an inflammatory cytokine.
(3). The device of paragraph 2, wherein the antibody or antigen-binding fragment thereof is specific to IL-6 or VEGF.
(4). The device of any of paragraphs 1-3, wherein the antibody or antigen-binding fragment thereof is attached to the polymeric microgel, optionally wherein the antibody or antigen-binding fragment thereof is attached to the polymeric microgel by a crosslinker, optionally wherein the crosslinker comprises aminopropyltrimethoxysilane (APTMS) or NHS-PEG-maleimide.
(5). The device of paragraph 4, wherein the tube comprises silicone and/or poly(dimethylsiloxane).

The devices disclosed herein relate to devices comprising a chamber component of an extracorporeal circuit, the chamber comprising a surface and an antibody or antigen-binding fragment thereof, a ligand, or a receptor. In some options, the surface comprises a polymer. In some options, the surface comprises the surface of a particle contained within the chamber. In certain options, the particle is a gel particle or a hydrogel particle. The particle may also be a microparticle or a nanoparticle in some cases. In certain options, the particle has an average surface area of at least about 5 m²/g, and/or an average pore volume of at least about 0.005 cm³/g. In some cases, the surface comprises a surface of a tube or a microfluidic device. In some options, the surface and antibody or antigen-binding fragment thereof, ligand, or receptor are contained within a carrier contained within the chamber.

In some options, the antibody or antigen-binding fragment thereof, ligand, or receptor is attached to the surface. In some cases, the antibody or antigen-binding fragment thereof, ligand, or receptor is attached to the surface by crosslinkers. In some options, the crosslinkers comprise aminopropyltrimethoxysilane (APTMS) or the heterobifunctional molecule N-hydroxylsuccinimide (NHS) polyethylene glycol (PEG) maleimide. In certain options, the polymer is an interpenetrating or semi-interpenetrating polymer network. In some options, the antibody or antigen-binding fragment thereof is specific to an inflammatory cytokine. In certain options, the antibody or antigen-binding fragment thereof is specific to IL-6. In some cases, the antibody or antigen-binding fragment thereof is specific to VEGF (including all of its isoforms).

Also disclosed herein are methods of targeted protein removal from a biological fluid of a subject in need thereof, comprising: contacting a sample of biological fluid taken from a subject with a surface or particle and an antibody or antigen-binding fragment thereof, a ligand, or a receptor that selectively binds to a protein suspected of being within the sample of biological fluid.

In some options, the biological fluid is blood, cerebrospinal fluid or a lymph fluid. In some options, the biological fluid is serum or plasma that has been separated from blood. In some options, the serum or plasma will be reconstituted with the original blood constituents and returned to the subject as filtered whole blood. In some options, methods further comprise reintroducing at least a portion of the sample of blood to the subject. In some options, the ligand, receptor, or antibody or antigen-binding fragment thereof is attached to the surface or particle. In some options, the blood is taken from the subject via an extracorporeal circuit.

Also disclosed herein are methods of targeted protein removal from the blood of a subject in need thereof, comprising: injecting into a subject a magnetically-susceptible particle comprising a surface and an antibody or antigen-binding fragment thereof, a ligand, or a receptor that selectively binds to a protein suspected of being within the blood of the subject; removing a sample of blood containing the magnetically-susceptible particle from the subject; and exposing the sample of blood containing the magnetically-susceptible particle to a magnetic field. In some options, blood is removed from the subject and magnetically-susceptible particles are added to the blood. The particles may bind to the targeted protein, the blood exposed to a magnetic field to remove at least some of the particles, and the blood can then be returned to the subject.

In some options, methods further comprise reintroducing at least a portion of the sample of blood to the subject. In some options, the act of removing the sample of blood comprises removing the sample of blood after a time at least sufficient to allow the magnetically-susceptible particle to circulate within the blood of the subject. In some options, the antibody or antigen-binding fragment thereof, ligand, or receptor is attached to the surface. In some options, the magnetic field is used to remove the magnetically-susceptible particle from the sample of blood. In some options, the magnetically-susceptible particle comprises a magnetically-susceptible core and a polymer at least partially surrounding the core. In some options, the magnetically-susceptible particle comprises iron.

The present disclosure is generally directed to an extracorporeal circuit comprising tubing comprises an antibody or antigen-binding fragment thereof positioned on a surface of the tubing. The present disclosure is also generally directed to an article comprising tubing comprises an antibody or antigen-binding fragment thereof positioned on a surface of the tubing. The present disclosure also relates to a method comprising passing a biological fluid removed from a subject through tubing comprising an antibody or antigen-binding fragment thereof positioned on a surface of the tubing, and returning at least a portion of the biological fluid to the subject.

The present disclosure, as well as various options thereof, will become more apparent in reference to the drawings and detailed description of the disclosure.

### BRIEF DESCRIPTION OF DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:

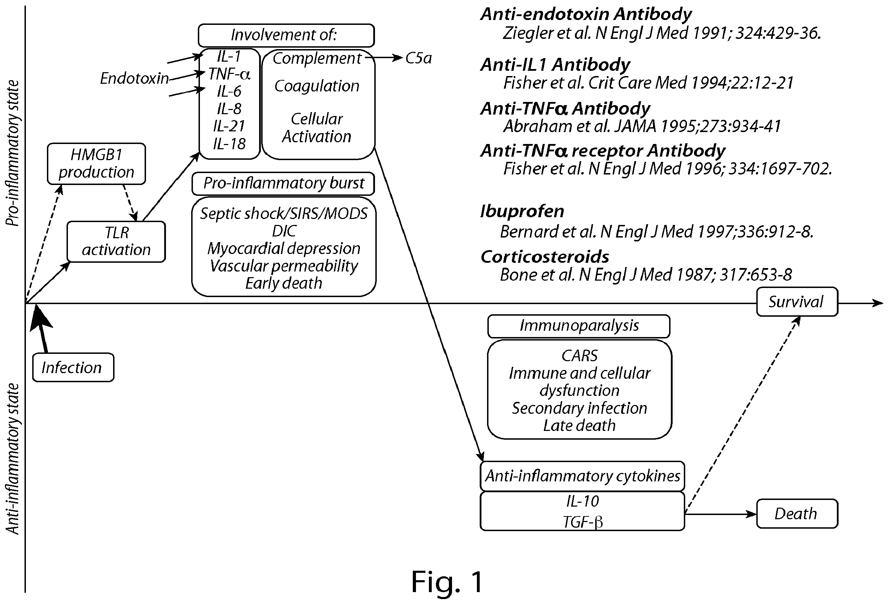

FIG. 1 provides a schematic of inflammatory and anti-inflammatory protein production during the progression of septic shock.
FIG. 2 provides a schematic depicting highly selective particles exhibiting antibodies throughout, in one option.
FIG. 3 provides a schematic depicting synthesis of a semi-interpenetrating polymer network functionalized with an antibody, in accordance with another option.
FIG. 4 provides a schematic representation of an experimental extracorporeal circuit driven by a peristaltic pump, in yet another option.
FIG. 5 provides a schematic diagram of a veno-venous extracorporeal circuit in a septic rat after cecal ligation and puncture, in still another option. Blood is drained from the femoral vein and returned via the jugular vein. Some or all of the entire length of tubing may comprise the active surface for protein removal.
FIG. 6 provides a schematic depicting synthesis of a polymeric surface functionalized with an antibody, in yet another option.
FIG. 7 provides a plot of cytokine concentrations in a biological fluid versus time following circulation of the biological fluid through an extracorporeal circuit in accordance with another option. VEGF is the targeted cytokine. IL-6 is the untargeted protein.

### DETAILED DESCRIPTION

The present disclosure generally relates to systems and methods for targeted removal of a substance or biomolecule such as a protein from a biological fluid, such as blood. In some cases, the blood may be withdrawn from a subject, treated, and returned to the subject. Previous techniques for removal of biological materials from blood, such as hemodialysis and plasmapheresis, were generally non-specific (i.e., they removed a multitude of proteins/toxins from the blood). By contrast, novel methods and devices described herein are capable of removing specific or single substances such as proteins from biological fluids such as blood in a specific manner. Such highly specific protein removal has a broad array of clinical applications, including treatment of inflammatory conditions and autoimmune diseases.

The present disclosure is not limited in its application to the details of construction and the arrangement of components set forth in the following description or illustrated in the drawings. The present disclosure is capable of other options and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

Also disclosed herein, the present disclosure relates to the use of an extracorporeal circuit to remove specific substances from biological fluid of a subject. For example, a biological fluid such as blood may be removed from the subject, treated in some fashion, and returned to the subject, i.e., the circuit allows for the return of the biological fluid back to the subject. It should be appreciated that any biological fluid of a subject can be compatible with the present disclosure. In some options, for example, the biological fluid is blood, cerebrospinal fluid or a lymph fluid.

It should also be appreciated that methods and compositions described herein can be applied to any substance that is targeted for removal from the biological fluid of a subject. In some options, for instance, the substance is a drug, a protein, a salt, hemoglobin, myoglobin, or a hormone.

In some non-limiting options, the substance to be removed from biological fluid is targeted using an antibody-antigen interaction, a ligand-receptor interaction, a drug-receptor interaction (e.g., gyrase sub-unit B and antibiotics such as coumermycin), a DNA aptamer, a chelating agent including a metal chelating agent (e.g., chelation of lead with D-Penicillamine, 2,3-Dimercaptosuccinic acid and chelation of mercury with 2,3-dimercapto-1-propanesulfonic acid), a molecular imprinted polymer (MIP) (e.g., for the removal of adrenergic hormones), a salt binding agent for the removal of salts (e.g., removal of potassium by carrageenan (1,4-linked a-D-galactose and 1,3 linked-b-D-galactose with a variable portion of sulfate groups) ions, a chiral interaction between a protein and a ligand, a synthetic DNA-ligand complex, a protein with a specific binding site (e.g., "lock and key" principle) and a ligand-cell interaction (e.g., through folic acid-folate receptor). In some options, the substance that is removed from the biological fluid is heparin or a drug.

In certain options, the substance to be removed from the biological fluid of a subject is an inflammatory mediator that drives inflammation, such as a cytokine. As used herein, a "cytokine" refers to a protein that is secreted by a cell of the immune system and that has an effect on other cells. Several non-limiting groups of cytokines include interleukins and interferons. Several non-limiting examples of interleukins include 1-18 (IL-1, IL-2, IL-3, IL-4, IL-5, IL-6, IL-7, IL-8, IL-9, IL-10, IL-11, IL-12, IL-13, IL-14, IL-15, IL-16, IL-17 and IL-18). IL-1 includes interleukin-1 alpha and interleukin-1 beta (IL-1 alpha and IL-1 beta). IL-5 is also known as eosinophil differentiation factor (EDF). IL-6 is also known as B-cell stimulatory factor-2 (BSF-2) and interferon beta-2. Several non-limiting examples of interferons include IFN-alpha (IFN-α), IFN-beta (IFN-β), IFN-omega (IFN-ω) and IFN-gamma (IFN-γ). Further examples of cytokines include TNF-alpha (TNF-α), TGF-beta-1 (TGF-β1), TGF-beta-2 (TGF-β2), TGF-beta-3 (TGF-β3) and vascular endothelial growth factor (VEGF).

Also disclosed herein, the present disclosure relates to modifying the abnormal immune response that is characteristic of many inflammatory diseases in a subject, e.g., in need thereof. In some options, a subject has an abnormal balance of inflammatory and anti-inflammatory cytokines, and the targeted protein removal targets one or more cytokines to alter the balance of inflammatory and anti-inflammatory cytokines in the subject. The subject can have a disorder affecting the immune system, such as an inflammatory disease or an autoimmune disease. Specific examples are discussed in more detail below.

In some options, the inflammatory disease is sepsis or septic shock. Sepsis is the leading cause of death in critically ill patients in the United States. Severe sepsis and septic shock are major health care problems, affecting millions of people worldwide each year and increasing in incidence. For example, sepsis develops in 750,000 people annually in the United States, resulting in death for more than 210,000 of them.

Septic shock may be caused by an unregulated immune response to a severe infection. The immune response is often biphasic, initially resulting in an inflammatory burst followed by immune suppression. The first phase is characterized by multi-organ failure, circulatory collapse, and death. Patients who survive the first phase go on to develop the compensatory anti-inflammatory response syndrome which is characterized by severe immune suppression and secondary infection. Many patients who develop septic shock succumb to secondary hospital acquired infection during the latter phase. Without wishing to be bound by any theory, sepsis is thought to represent an uncontrolled inflammatory response elicited by infection, based on animal studies that documented "cytokine storms" when septic shock was induced by injection of bacteria, lipopolysaccharide administration, or cecal ligation and puncture (CLP). Cytokines such as tissue necrosis factor alpha (TNF-α), interleukin-1-beta (IL-1β), IL-6, VEGF, and many more have been implicated. However, cytokines also have beneficial effects in sepsis. In addition to being an essential part of the immune response, some of them possess anti-inflammatory properties (e.g., TGF-beta (TGF-β), IL-10).

Various options of the disclosure relate to removal of specific cytokines from the blood of a subject. In someoptions, the cytokine that is targeted for removal from the blood of a subject is IL-6. IL-6 is biomarker of the activation status of the cytokine network and is known to reflect the influence of several cytokines. IL-6 is a pleiotropic cytokine, primarily involved in the regulation of immune and inflammatory responses. IL-6 can be generated by T- and B-lymphocytes, monocytes/macrophages, fibroblasts, vascular smooth muscle cells, endothelial cells, and even mesangial and tubular epithelial cells of the kidney. IL-6 has many biological properties that result in the up-regulation of a variety of effects; for instance, tissue factor and matrix-degrading enzyme production, C-reactive protein and fibrinogen formation in hepatocytes, and also forms part of a positive feedback loop for tissue necrosis factor-alpha. Circulating IL-6 levels are reproducibly detectable in patients with sepsis, and higher concentrations portend a poor outcome. Accordingly, in certain options of the disclosure, antibodies directed to the removal of IL-6 are used.

In some options, the cytokine that is targeted for removal is VEGF. VEGF is a signal protein that stimulates vasculogenesis and angiogenesis; it has been reported to stimulate permeability, proliferation, migration, and survival of endothelial cells and to contribute to inflammation and coagulation. Sepsis has been associated with elevated expression and circulating levels of VEGF. It has been suggested that elevated VEGF levels in sepsis patients may sensitize endothelial cells to the effects of low TNF-alpha (TNF-α) and promote endothelial permeability, thereby contributing to morbidity and mortality in sepsis. In certain options of the disclosure, antibodies directed to the removal of VEGF were therefore used.

Thus, certain options of the present disclosure are generally directed to modification of biological fluids, e.g., extracorporeal blood modification, using an extracorporeal circuit, i.e., a circuit for fluid that exits the subject for treatment, and returns the fluid to the subject, e.g., on a continuous basis. In some cases, a biological fluid such as blood may be removed from a subject and exposed to a molecule such as an antibody or ligand, which can be used to specifically remove one or more substances such as proteins (for example IL-6 or VEGF), or other species, from the blood. For example, the biological fluid, such as blood, can be modified using an extracorporeal circuit that may contain one or more surfaces and a suitable agent such as an antibody. For example, one or more agents such as antibodies or ligands may be anchored to a particle or to circuitry surfaces, such that the antibody is the functional component of a system that is used to target a species, such as a cytokine. The surface may comprise a polymer, such as polymerized dopamine, poly(dimethylsiloxane), or other polymers as discussed herein. The antibody/surface system can be, for example: (1) for surface modification of extracorporeal circuits and/or (2) creation of ferromagnetic (rendering them magnetically-susceptible) particles, such as nanoparticles, with superparamagnetic cores, among other applications.

For example, for surface modification of an extracorporeal circuit, a chamber component of an extracorporeal circuit may comprise a surface and an antibody or antigen-binding fragment thereof in one option. In another option, the chamber component of an extracorporeal circuit may comprise a particle and an antibody or antigen-binding fragment. The chamber may include a compartment or space through which the blood of a subject passes during extracorporeal circulation. For example, a surface within an extracorporeal circuit can be at least partially coated with an antibody, thereby rendering it capable of scavenging cytokines from the blood that circulates through the system.

The chamber may take a variety of forms in different options of the disclosure, but typically contains at least one surface and an antibody or antigen-binding fragment thereof, a ligand, or a receptor. The surface may be, for example, a surface defining a wall of the chamber, an internal surface contained within a chamber, or a surface of a particle. In one option, the chamber may be a well-defined rectangular chamber. In some options, however, the chamber may comprise a tube, e.g., at least a portion of the tube is coated with the polymer or particle and the antibody. In some options, the chamber may comprise a microfluidic device, e.g., at least a portion of the microfluidic channels is coated with the polymer or particle and the antibody.

As a non-limiting example of an extracorporeal circuit, in one set of options, a fluid such as blood is withdrawn from a subject (e.g., a human), passed through a length of tubing, and returned to the subject. The tubing may comprise a surface as part of the tubing that defines a surface that is able to extract one or more substances such as proteins (or other species discussed herein) from the blood. For example, at least a portion of the tubing may comprise a surface which is at least partially coated with an antibody as discussed herein. For instance, at least about 10%, at least about 20%, at least about 30%, at least about 40%, at least about 50%, at least about 60%, at least about 70%, at least about 80%, at least about 90%, or about 100% of the inner surface of the tubing may be coated. As fluid passes through the tubing, at least some of the antibody (or antibody or antigen-binding fragment thereof, or ligand or receptor, etc.) on the surface may be able to extract one or more substances such as proteins (or other species discussed herein) from the blood, e.g., through binding of the proteins.

In some options, the chamber or tubing comprises or contains a carrier that carries the surface and antibody or antigen-binding fragment thereof, ligand, or receptor. A carrier may, for example, comprise solid particles, e.g., comprising polymer, ceramic, metal, and/or other materials. The carrier may have any suitable shape, e.g., as particles, fibers, ribbons, etc. The carrier may be relatively inert, e.g., the carrier is used to increase the surface area within the chamber. The carrier may be contained within the chamber, and/or be attached to a surface within the chamber. In some options, the surface area of a chamber is increased to increase the amount of surface and antibody exposure, such as by the incorporation of carriers, baffles, plates, grills, particles, or other suitable surfaces within the chamber. In some options, a biological fluid such as blood is removed from a subject and modified through interaction with a surface, and then at least a portion of the biological fluid is reintroduced to the subject.

However, in another set of options, particles containing antibodies may be injected into a subject, allowed to bind to one or more proteins or other species within the subject, and then removed, thereby causing the removal of one or more substances such as proteins (for example IL-6 or VEGF), or other species, from the blood, e.g., via an extracorporeal circuit. For example, one set of options is generally directed to magnetically-susceptible particles, such as nanoparticles, that comprise a surface and an antibody or antigen-binding fragment thereof that selectively binds to a protein suspected of being within the blood of a subject. In some options, the magnetically-susceptible particles are injected into a subject and allowed to circulate. After sufficient time to bind the protein (or other species), the magnetically-susceptible particles are removed from the subject. For example, blood from the subject may be removed via an extracorporeal circuit that is magnetized such that the particles (now bound to the protein or other species) are extracted from the blood circulating in the extracorporeal circuit. In some options, the particles are biodegradable and/or biocompatible. In some options, the particles are allowed to circulate in the biological fluid of a subject for up to approximately 12 hours or up to approximately 24 hours.

Magnetically-susceptible particles, such as ferromagnetic particles, may be selected in some cases so as to possess a greater density than other elements of the blood. Accordingly, in some options, these particles are removed from the blood by centrifugation, magnetization, or a combination of these and/or other techniques. In certain options, an aliquot of blood is removed from a subject, mixed with a predetermined amount of ferromagnetic particles under conditions that allow optimal binding to the desired product for removal. Upon formation of the particle:blood constituent complex, for instance, the sample can be centrifuged and/or magnetized to remove the target blood component.

The extracorporeal circuits discussed above may be used solely to remove a substance such as a protein or other species from a biological fluid, or the extracorporeal circuit may be used in conjunction with other systems and/or methods. For example, in some options, a polymer or particle and antibody are incorporated into a cardiopulmonary bypass circuit during heart surgery to prevent (or at least control) an inflammatory response.

### Polymers/Substrates

A surface comprising a polymer as disclosed herein may be the surface of a substrate as discussed herein. The substrate may be used in extracting specific substances such as proteins (or other species) from a fluid such as blood. In some cases, the substrates may be formed from or include the polymer. In other cases, the substrate may be formed from a material to which the polymer is coated thereon. In some cases, the substrate may be present as particles such as microgel particles. Non-limiting examples of substrates and polymers compatible with the present disclosure are further described in PCT Application No. PCT/US2012/026008, filed on February 22, 2012, and from Mizrahi et al. (2011) Advanced Materials 23:H258-H262.

The substrate may have any suitable shape. For example, the substrate may be formed as particles, as a planar substrate, or the like. In one set of options, the substrate is polymeric. For example, the substrate may include a polymer such as poly(acrylamide), e.g., formed through the polymerization of acrylamide and a suitable metal-chelating moiety, as discussed below. For instance, acrylamide may be polymerized to form poly(acrylamide) upon exposure to ammonium persulfate, methylenebisacrylamide, and/or N,N,N',N'-tetramethylethylendiamine ("TEMED"). In some cases, the polymerization may occur within an emulsion, e.g., to form particles. For example, an emulsion may be formed where monomers are present within discrete droplets (e.g., in an aqueous environment) contained within a continuous phase (e.g., an organic or "oil" environment), and polymerization induced within the discrete droplets to form polymeric particles.

Other examples of suitable polymers that can be used in the substrate include, but are not limited to, poly(styrene), poly(propylene), poly(ethylene), poly(dimethylsiloxane), agarose, and the like, e.g., in addition to and/or instead of poly(acrylamide). Still other examples include polyethylene, polystyrene, silicone, polyfluoroethylene, polyacrylic acid, a polyamide (e.g., nylon), polycarbonate, polysulfone, polyurethane, polybutadiene, polybutylene, polyethersulfone, polyetherimide, polyphenylene oxide, polymethylpentene, polyvinylchloride, polyvinylidene chloride, polyphthalamide, polyphenylene sulfide, polyester, polyetheretherketone, polyimide, polymethylmethacylate and/or polypropylene. Polymeric particles or other substrates formed using these polymers may be formed using techniques known to those of ordinary skill in the art.

The substrate may have any suitable shape. For example, the substrate may be present as particles, or as the surface of a chamber or a tube. In some options, the substrate may have a relatively high surface area. For example, the substrate having the attached antibody or antigen-binding fragment thereof (or a ligand or a receptor, etc.) may have a surface area of attachment of the antibodies, etc. of at least about 0.01 m², at least about 0.02 m², at least about 0.03 m², at least about 0.05 m², at least about 0.1 m², at least about 0.2 m², at least about 0.3 m², at least about 0.5 m², at least about 1 m², at least about 2 m², at least about 3 m², at least about 5 m², or at least about 10 m². In another set of options, the substrate may have an average surface area of at least about 5 m²/g, at least about 7 m²/g, or at least about 10 m²/g. In some options, the substrate may have an average pore volume of at least about 0.005 cm³/g, at least about 0.01 cm³/g, or at least about 0.02 cm³/g.

As mentioned, the substrate may take the form of one or more particles. In some cases, the particles may include microparticles and/or nanoparticles. A "microparticle" is a particle having an average diameter on the order of micrometers (i.e., between about 1 micrometer and about 1 mm), while a "nanoparticle" is a particle having an average diameter on the order of nanometers (i.e., between about 1 nm and about 1 micrometer). As additional examples, the particles may have an average diameter of less than about 5 mm or 2 mm, or less than about 1 mm, or less than about 500 micrometers, less than about 200 micrometers, less than about 100 micrometers, less than about 80 micrometers, less than about 60 micrometers, less than about 50 micrometers, less than about 40 micrometers, less than about 30 micrometers, less than about 25 micrometers, less than about 10 micrometers, less than about 3 micrometers, less than about 1 micrometer, less than about 300 nm, less than about 100 nm, less than about 30 nm, or less than about 10 nm. In some options, the particle may have an average diameter of at least about 1 micrometer or at least about 10 micrometers. Also, the particles may be spherical or non-spherical. If the particle is non-spherical, the particle may have a shape of, for instance, an ellipsoid, a cube, a fiber, a tube, a rod, or an irregular shape. The average diameter of a non-spherical particle is the diameter of a perfect sphere having the same volume as the non-spherical particle.

In certain options, the substrate may be a gel. Non-limiting examples of gels include poly(acrylamide) gel or agarose gel, or other gel materials such as those describe herein. For example, if the substrate is a particle, then the substrate may take the form of microgel particles or gel microparticles. In some options, the gel particles may be collected together to form a gel material or a "microgel." A gel typically is relatively solid or jelly-like, and may include a cross-linked polymer to form its structure. In some cases, the gel may be a hydrogel, e.g., a gel that contains water.

The substrate may be porous, in certain options of the disclosure. In some options, the substrate may have a relatively high surface area, for example, having an average surface area of at least about 5 m²/g, at least about 7 m²/g, or at least about 10 m²/g. In some options, the substrate may have an average pore volume of at least about 0.005 cm³/g, at least about 0.01 cm³/g, or at least about 0.02 cm³/g. In other options, the substrate may have an average pore width of at least about 5 nm, at least about 7 nm, or at least about 8 nm. Such porosities and dimensions may be determined using techniques known to those of ordinary skill in the art, for example, TEM, SEM, BET, or the like. The porosity may be created, for example, due to the nature of the polymer (e.g., certain gel polymers such as those described herein typically will form relatively porous structures), or the porosity may be induced by adding another material to the substrate that can be removed, thereby creating porosity within the substrate. For example, salts or other species that can be subsequently dissolved may be incorporated within the substrate.

In one set of options, the substrate takes the form of one or more magnetically-susceptible particles, e.g., having dimensions, etc. as described herein. In some cases, a magnetically-susceptible particle comprises at least one material that is magnetically-susceptible, e.g., such that the particle may be manipulated using a suitable magnetic field. For example, the particle may comprise a ferromagnetic material or a superparamagnetic material, e.g., iron, cobalt, nickel, or the like. In some cases, the magnetically-susceptible portion of the particle is a core of the particle, coated with polymer and/or an antibody such as is discussed herein.

In various options, the polymer may be an interpenetrating or a semi-interpenetrating polymer, such as a semi-interpenetrating polymer network with an antibody affixed to polymer subunits throughout the network. An "interpenetrating polymer network" or an "IPN" typically comprises a polymeric material comprising two or more networks of two or more polymers (which can include copolymers) at least two different polymers of which are at least partially interlaced with respect to each other on a molecular scale, but not covalently bonded to each other. These polymer networks cannot be separated, even theoretically, unless one or more covalent bonds are broken. Thus, a mixture of two or more pre-formed polymers (e.g., as in a mixture or a blend) is not an interpenetrating polymer network. Specific non-limiting examples of an interpenetrating network include [net-poly(styrene-stat-butadiene)]-ipn-[net-poly(ethyl acrylate), polyHEMA-ipn-polyurethane, or polyHEMA-ipn-polysiloxane, where "polyHEMA" is poly(2-hydroxyethylmethacrylate)].

Those of ordinary skill in the art are able to prepare IPNs using suitable techniques, for example, by blending different polymer precursors which have the ability under set conditions to react to form two or more different interpenetrating polymers that do not covalently bind to each other, by forming a first polymer and allowing a precursor of a second polymer to diffuse into the first polymer in an interpenetrating manner and to react to form the second polymer under conditions that do not promote binding between the first and second polymer, by mixing two or more different families of monomers, each capable of polymerizing via different mechanisms (for example, a radical reaction and a condensation reaction) but are not capable of reacting with each other and sequentially or simultaneously polymerizing the monomers using heat and/or UV light, by blending two or more linear or branched polymers with at least one polymer having pendant reactant groups and subsequently adding a chain extender to cross-link each of the polymers into separate networks, and/or by proceeding with a multi-stage polymerization process including a first polymer network that is partially polymerized to allow for high swellability and/or easy diffusion of a second polymer precursor, allowing the second polymer precursor to penetrate the first polymer network, and thereafter polymerizing both polymer networks, etc.

A "semi-interpenetrating polymer network" or a "SIPN," typically comprises a polymeric material comprising a combination of at least one polymer network (which may include a copolymer or copolymers) and one or more linear or branched polymer(s), characterized by the penetration, on a molecular scale, of at least one of the networks by at least some of the linear or branched macromolecules. Semi-interpenetrating polymer networks can also be defined as similar to interpenetrating polymer networks, but distinguished in that a constituent linear or branched polymer(s) can be (at least in theory) separated from the polymer network(s) without breaking any covalent bonds. However, such separation would require "unthreading" of the linear or branched polymer(s) from the polymer network, an impossibility for most such systems. A specific non-limiting example of a semi-interpenetrating network is (net-polystyrene)-sipn-poly(vinyl chloride).

As further examples, interpenetrating and/or semi-interpenetrating polymer networks can also be formed from combinations of polyethylenes (e.g., tetrafluoroethylenes) and silicone polymers, or acrylates (e.g., methylmethacrylates) and urethanes and/or ureas. Those of ordinary skill in the art are able to form SIPNs by known techniques, for example, using techniques such as those described above with reference to interpenetrating networks, or by allowing a solution of a linear or branch polymer to diffuse into a polymer network, by blending two or more linear or branched polymer networks with at least one polymer having pendant reactant groups and subsequently adding a chain extender to cross-link one of the polymer networks, by proceeding with a multi-stage polymerization process including a first polymer network that is partially polymerized to allow for high swellability and/or easy diffusion of a second polymer precursor therein, and thereafter polymerizing the first polymer network, etc.

It is to be understood that a material of the disclosurecan include an interpenetrating network which includes a semi-interpenetrating component or components; e.g., a material of the disclosure can include two polymer networks that are interpenetrating, and a third that is semi-interpenetrating with respect to one or both of the two interpenetrating networks (and can include any number of additional interpenetrating or semi-interpenetrating components). An interpenetrating network can include a semi-interpenetrating component or components.

Those of ordinary skill in the art will know of suitable techniques for identifying and/or determining interpenetrating and/or semi-interpenetrating polymer networks. Examples of characterization techniques for the identification and/or determination of a polymer from an interpenetrating and/or semi-interpenetrating polymer network include techniques that allow for the observation of microdomains, as opposed to polymers or polymer blends that separate into macrodomains. Example of such techniques include, but are not limited to, differential scanning calorimetry (DSC), which allows the identification and/or determination of multiple glass transition temperatures (i.e., in an interpenetrating and/or semi-interpenetrating polymer network, each of the polymers comprising the network may have different glass transition temperatures); transmission electron microscopy (TEM), which allows for the microscopic identification of the microdomains of the interpenetrating and/or semi-interpenetrating polymer networks; CP-MAS solid NMR of ¹³C or ²⁹Si, which allows visualization of cross-link points within the interpenetrating and/or semi-interpenetrating networks in polymers having cross-link points; small angle neutron scattering (SANS) techniques, which allows the visualization of domains in a sample; differential mechanical analysis (DMA), which allow the modulus of each polymer within the interpenetrating and/or semi-interpenetrating polymer network to be determined; or the like.

It should be appreciated that antibodies and antigen-binding fragments thereof associated with certain options of the disclosure can be complexed or otherwise associated with a polymeric system according to any method known in the art. In some options, the polymeric system is activated through surface oxidation, such as by plasma oxidation or acid oxidation. The activated polymeric system may be aminated in some cases by an aminosilane, such as aminopropyltrimethoxysilane (APTMS) or aminopropyltriethoxysilane (APTES). In some options, the antibody is attached to polymerized dopamine by a histidine linker. In some options, the histidine linker is 3, 4, 5, 6, 7, 8, 9, 10, 11, 12, 13, 14, 15, or more than 15 histidines. In certain options, the histidine linker is formed from 6-8 histidines. In some options , the antibody is attached to the polymeric system by an APTMS-PEG-maleimide linker. Other examples of suitable types of crosslinkers that are commercially available and known in the art include, but are not limited to, haloacetyls and pyridyldithiols. In some options, the antibody is chemically modified, such as by coupling it with N-succinimidylacrylate (NSA) or with a furan functionality (e.g., for a Diels-Alder reaction). In some options, copolymerization is achieved by combining the modified antibody with acrylamide, aqueous ammonium persulphate and N,N,N',N'-tetramethylethylenediamine (TEMED).

It should be appreciated that a surface, such as a surface of a chamber component of an extracorporeal circuit, can be modified with a polymer and/or hydrogel as disclosed herein according to any method known in the art. In some options, methods of functionalizing a surface with polymeric microgel films are derived from methods based on plasma-induced graft polymerization of poly acrylic acid, such as is described in Singh et al. (2007) Biomacromolecules 8(10):3271-5. In someoptions, a photoaffinity label, viz., aminobenzophenone is introduced onto the surface.

In one set of options, the substrate may be positively or negatively charged, e.g., to facilitate separation of proteins, or other analytes. For example, an analyte may be positively charged and a negatively charged substrate may facilitate attraction of the analyte. For instance, a protein may be positively charged due to residues such as glutamine or asparagine on the protein, which may be attracted to negatively charged particles or other substrates. As another example, an analyte may be negatively charged, and a positively charged particle may facilitate attraction of the analyte. For instance, a nucleic acid such as DNA or RNA may be negatively charged, and the nucleic acid may be attracted to positively charged particles or other substrates. In one set of options, an acrylic acid or other monomer producing negatively charged residues may be incorporated into the polymer or otherwise added to the substrate to impart a negative charge on the substrate. In another set of options, a monomer producing positively charged residues (e.g., ethylenimine), may be used to impart a positive charge on a substrate, e.g., via incorporation into the polymer or other addition to the substrate.

The substrate may also comprise a metal-chelating moiety, for example, EDTA (ethylenediaminetetraacetic acid) or NTA (nitrilotriacetic acid), or derivatives thereof, to which metal ions, including divalent metal ions, are able to bind. Other non-limiting examples of metal-chelating moieties include various polyamino carboxylic acid such as Fura-2, iminodiacetic acid, diethylene triamine pentaacetic acid (DTPA), ethylene glycol tetraacetic acid (EGTA), 1,2-bis(o-aminophenoxy)ethane-N,N,N',N'-tetraacetic acid (BAPTA), 1,4,7,10-tetraazacyclododecane-1,4,7,10-tetraacetic acid (DOTA), or the like, and derivatives thereof. The metal-chelating moiety may be one which is able to bind or complex with metal ions, such as divalent metal ions. Non-limiting examples of ions that can be chelated by metal-chelating moieties include nickel, cobalt, calcium, iron, or the like. As a specific non-limiting example, a metal-chelating moiety may bind to nickel ions, so that a substrate containing the metal-chelating moiety may also contain nickel ions distributed within the substrate.

In some options, the metal-chelating moiety may be incorporated into the polymeric structure of a substrate. The metal-chelating moiety may be present as a monomer as various monomers are polymerized and/or cross-linked to form a polymeric substrate, e.g., forming a copolymer or an interpenetrating or semi-interpenetrating polymer network. For example, the metal-chelating moiety may be incorporated in a polymer as a monomer such that when the polymer is formed, one of the monomers or residues within the polymer is the metal-chelating moiety. As a specific non-limiting example, an NTA derivative such as 2,20-(5-acrylamido-1-carboxypentylazanediyl)diacetic acid may be used, which forms NTA residues when incorporated within a polymer.

In some cases, the metal-chelating moiety may be distributed substantially evenly throughout the substrate. For example, the concentration of the metal-chelating moiety on the surface of the substrate and in the bulk or the center of the substrate may be substantially the same. For instance, the difference in concentration of the metal-chelating moiety between the surface of the substrate and the bulk or center of the substrate may be no more than about 40%, no more than about 35%, no more than about 30%, no more than about 25%, no more than about 20%, no more than about 15%, no more than about 10%, or no more than about 5%, where the percentage is taken relative to the average of these concentrations on the surface and in the bulk or center of the substrate.

The distribution of the metal-chelating moiety within the substrate may be relatively uniform, for example, if the metal-chelating moiety is formed as an integral part of the substrate as the substrate is formed. For instance, the metal-chelating moiety may be incorporated within a polymer as a monomer within the polymer, thus resulting in a relatively uniform distribution of the metal-chelating moiety within the polymeric substrate.

In some options, metal ions may be allowed to become distributed within the substrate, e.g., by exposing or the substrate to a fluid containing the metal ions, for example, such that the ions are able to penetrate the substrate via diffusion or other forces (e.g., charge attraction). In some cases, the substrate may be immersed in the fluid. The metal ions may become distributed within the substrate uniformly or non-uniformly, e.g., depending on the length of exposure. For instance, if the metal-chelating moiety is distributed relatively uniformly within the substrate, then metal ions attracted to the metal-chelating moiety may likewise become relatively uniformly within the substrate.

### Antibodies

The disclosure may relate to antibodies that bind selectively to proteins suspected of being within the blood of a subject. An antibody is a protein that typically includes at least two heavy (H) chains and two light (L) chains inter- by disulfide bonds. Each heavy chain is comprised of a heavy chain variable region (abbreviated herein as HCVR or V_{H}) and a heavy chain constant region. The heavy chain constant region is comprised of three domains, C_{H}1, C_{H}2 and C_{H}3. Each light chain is comprised of a light chain variable region (abbreviated herein as LCVR or V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antigen-binding fragment" of an antibody as used herein, refers to one or more portions of an antibody that retain the ability to specifically bind to an antigen (e.g., a cytokine). It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C_{H}1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment which consists of a V_{H} domain or the variable domain of a heavy-chain antibody, such as a camelid heavy-chain antibody (e.g. V_{HH}); (vi) an isolated complementarity determining region (CDR); and (vii) polypeptide constructs comprising the antigen-binding fragments of (i) - (vi). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv). Such single chain antibodies are also intended to be encompassed within the term "antigen-binding portion" of an antibody. These antibody fragments are obtained using conventional procedures, such as proteolytic fragmentation procedures, expression of recombinant nucleic acids, or the like. The fragments are screened for utility in the same manner as are intact antibodies.

Isolated antibodies of the disclosure encompass various antibody isotypes, such as IgG1, IgG2, IgG3, IgG4, IgM, IgA1, IgA2, IgAsec, IgD, IgE. As used herein, "isotype" refers to the antibody class (e.g., IgM or IgG1) that is encoded by heavy chain constant region genes. Antibodies as disclosed herein can be full length or can include only an antigen-binding fragment such as the antibody constant and/or variable domain of IgG1, IgG2, IgG3, IgG4, IgM, IgA1, IgA2, IgAsec, IgD or IgE or could consist of a Fab fragment, a F(ab')₂ fragment, and a Fv fragment.

Antibodies of the present disclosure can be polyclonal, monoclonal, or a mixture of polyclonal and monoclonal antibodies. Antibodies of the disclosure can be produced by methods disclosed herein or by a variety of techniques known in the art, and many such antibodies can readily be obtained commercially.

The present disclosure encompasses both polyclonal and monoclonal antibodies, including antibodies prepared using techniques that are known in the art. A monoclonal antibody typically refers to a preparation of antibody molecules of single molecular composition. A monoclonal antibody may display a single binding specificity and affinity for a particular epitope. A monoclonal antibody may display a single binding specificity and affinity for a particular epitope. The polyclonal antibody typically refers to a preparation of antibody molecules that comprises a mixture of antibodies active that specifically bind a specific antigen.

A process of monoclonal antibody production may include obtaining immune somatic cells with the potential for producing antibody, in particular B lymphocytes, which have been previously immunized with the antigen of interest either *in vivo* or *in vitro* and that are suitable for fusion with a B-cell myeloma line. Mammalian lymphocytes typically are immunized by *in vivo* immunization of the animal (e.g., a mouse) with the desired protein or polypeptide, e.g., a cytokine. Such immunizations are repeated as necessary at intervals of up to several weeks to obtain a sufficient titer of antibodies. Once immunized, animals can be used as a source of antibody-producing lymphocytes which can be cloned and recombinantly expressed, as discussed further below. Following the last antigen boost, the animals are sacrificed and spleen cells removed. Mouse lymphocytes give a higher percentage of stable fusions with the mouse myeloma lines described herein. Of these, the BALB/c mouse is preferred. However, other mouse strains, rat, rabbit, hamster, sheep, goats, camels, llamas, frogs, etc. may also be used as hosts for preparing antibody-producing cells. Mouse strains that have human immunoglobulin genes inserted in the genome (and which cannot produce mouse immunoglobulins) can also be used. Examples include the HuMAb mouse strains produced by Medarex/GenPharm International, and the XenoMouse strains produced by Abgenix. Such mice produce fully human immunoglobulin molecules in response to immunization.

Those antibody-producing cells that are in the dividing plasmablast stage fuse preferentially. Somatic cells may be obtained from the lymph nodes, spleens and peripheral blood of antigen-primed animals, and the lymphatic cells of choice depend to a large extent on their empirical usefulness in the particular fusion system. The antibody-secreting lymphocytes are then fused with (mouse) B cell myeloma cells or transformed cells, which are capable of replicating indefinitely in cell culture, thereby producing an immortal, immunoglobulin-secreting cell line. The resulting fused cells, or hybridomas, are cultured, and the resulting colonies screened for the production of the desired monoclonal antibodies. Colonies producing such antibodies are cloned, and grown either *in vivo* or *in vitro* to produce large quantities of antibody.

Myeloma cell lines suited for use in hybridoma-producing fusion procedures preferably are non-antibody-producing, have high fusion efficiency, and enzyme deficiencies that render them incapable of growing in certain selective media which support the growth of the desired hybridomas. Examples of such myeloma cell lines that may be used for the production of fused cell lines include, but are not limited to Ag8, P3-X63/Ag8, X63-Ag8.653, NS1/1.Ag 4.1, Sp2/0-Ag14, FO, NSO/U, MPC-11, MPC11-X45-GTG 1.7, S194/5XX0 Bul, all derived from mice; R210.RCY3, Y3-Ag 1.2.3, IR983F and 4B210 derived from rats and U-266, GM1500-GRG2, LICR-LON-HMy2, UC729-6, all derived from humans. Those of ordinary skill in the art will be aware of numerous routine methods to produce monoclonal antibodies.

Fusion with mammalian myeloma cells or other fusion partners capable of replicating indefinitely in cell culture is effected by standard and well-known techniques, for example, by using polyethylene glycol ("PEG") or other fusing agents.

Methods of raising polyclonal antibodies are well known to those of ordinary skill in the art. As a non-limiting example, polyclonal antibodies may be raised by administering a polypeptide subcutaneously to New Zealand white rabbits which have first been bled to obtain pre-immune serum. The polypeptide can be inoculated with (e.g., injected at) a total volume of 100 microliters per site at six different sites, typically with one or more adjuvants. The rabbits are then bled two weeks after the first injection and periodically boosted with the same antigen three times every six weeks. A sample of serum is collected 10 days after each boost. Polyclonal antibodies are recovered from the serum, preferably by affinity chromatography using acetylated cytochrome to capture the antibody. Those of ordinary skill in the art will be aware of numerous routine methods to produce polyclonal antibodies.

In other options, antibodies may be recombinant antibodies. Recombinant antibodies generally include antibodies that are prepared, expressed, created or isolated by recombinant means, such as antibodies isolated from an animal (e.g., a mouse) that is transgenic for another species' immunoglobulin genes, genetically engineered antibodies, antibodies expressed using a recombinant expression vector transfected into a host cell, antibodies isolated from a recombinant, combinatorial antibody library, or antibodies prepared, expressed, created or isolated by any other means that involves splicing of immunoglobulin gene sequences to other DNA sequences.

Antibodies or antigen-binding fragments of the disclosure are, preferably, isolated. In some cases, the isolated antibody is not present in an organism that endogenously produces the antibody, i.e., the antibody has been isolated from the organism. In some cases, isolated antibodies and antigen-binding fragments thereof refer to an antibody (or antigen-binding fragment thereof) that is substantially free of other antibodies (or antigen-binding fragments) having different antigenic specificities. An isolated antibody that specifically binds to an epitope, isoform or variant of a polypeptide (e.g., a cytokine) may, however, have cross-reactivity to other related antigens, e.g., a mutant form of the cytokine, or a polypeptide from other species (e.g., homologs in other species). Moreover, an isolated antibody (or antigen-binding fragment thereof) may be substantially free of other cellular material and/or chemicals.

Antibodies of the disclosure include, but are not limited to antibodies that specifically bind to a cytokine. As used herein, "selective binding" refers to antibody binding to a predetermined antigen with a preference that enables the antibody to be used to distinguish the antigen from others. In some options, the antibody binds to a single protein and is able to distinguish that protein from all other proteins. In other options, the antibody binds to several different related proteins and is able to distinguish those proteins from all other proteins.

In some options, multiple antibodies, such as multiple different monoclonal antibodies, are incorporated into the polymer system at any given time, resulting in an extracorporeal circuit that is capable of removing more than one cytokine at a time. In so doing, the highly specific nature of the system is preserved and yet expanded to remove groups of proteins in a fashion never before described.

In some options, an antibody or antigen-binding fragment thereof, of the invention, can specifically bind to an antigen with sub-nanomolar affinity. The dissociation constants can be about 1 x 10⁻⁶, 1 x 10⁻⁷, 1 x 10⁻⁸, 1 x 10⁻⁹ M or less, preferably about 1 x 10⁻¹⁰ M or less, more preferably 1 x 10⁻¹¹M or less. In a particular option the binding affinity is less than about 5 x 10⁻¹⁰M.

Also disclosed herein, an antibody or antigen-binding fragment thereof may bind to a conformational epitope of a polypeptide such as a cytokine. To determine if the selected antibodies bind to conformational epitopes, each antibody can be tested in assays using native protein (e.g., non-denaturing immunoprecipitation, flow cytometric analysis of cell surface binding) and denatured protein (e.g., Western blot, immunoprecipitation of denatured proteins). A comparison of the results will indicate whether the antibodies bind conformational epitopes. Antibodies that bind to native protein but not denatured protein are those antibodies that bind conformational epitopes, and are preferred antibodies.

In some options, a ligand, rather than an antibody, is used for selective binding to a substance such as a protein in a biological fluid such as blood.

An antibody or antigen-binding fragment thereof of the disclosure can be linked to a detectable label. A detectable label of the disclosure may be attached to antibodies or antigen-binding fragments thereof of the invention by standard protocols known in the art. In some options, the detectable labels may be covalently attached to an antibody or antigen-binding fragment thereof of the disclosure. The covalent binding can be achieved either by direct condensation of existing side chains or by the incorporation of external bridging moieties. Many bivalent or polyvalent agents are useful in coupling protein molecules to other proteins, polypeptides or amine functions, etc. For example, the literature is replete with coupling agents such as carbodiimides, diisocyanates, glutaraldehyde, and diazobenzenes. This list is not intended to be exhaustive of the various coupling agents known in the art but, rather, is exemplary of the more common coupling agents.

### Treatment

The methods disclosed herein are useful in some options for treating a subject in need thereof. In some options, a subject in need thereof can be a subject who has an inflammatory disease or an autoimmune disease. For example, a subject in need thereof can be a subject who has sepsis or septic shock, acute respiratory distress syndrome (ARDS), systemic inflammatory response syndrome (SIRS) related to cardiopulmonary bypass, myasthenia gravis, etc. In other options, a subject in need thereof can be a subject who has a neurodegenerative disease, such as Alzheimer's disease. In its broadest sense, the terms "treatment" or "to treat" refer to both therapeutic and prophylactic treatments. If the subject in need of treatment is experiencing a condition (i.e., has or is having a particular condition), then "treating the condition" refers to ameliorating, reducing or eliminating one or more symptoms associated with the disorder or the severity of the disease or preventing any further progression of the disease. If the subject in need of treatment is one who is at risk of having a condition, then treating the subject refers to reducing the risk of the subject having the condition or preventing the subject from developing the condition.

A subject as used herein means a human or other vertebrate animal or mammal including, but not limited to, a dog, cat, horse, cow, pig, sheep, goat, rodent, bird, and primate, e.g., monkey.

The term "effective amount" refers to the amount necessary or sufficient to realize a desired biologic effect. For example, an effective amount of a polymer or particle and an agent such as an antibody or antigen-binding fragment thereof associated with the disclosure may be that amount sufficient to ameliorate one or more symptoms of a disease such as an inflammatory disease or autoimmune disease. Combined with the teachings provided herein, by weighing factors such as potency, relative bioavailability, patient body weight, severity of adverse side-effects and preferred mode of administration, an effective prophylactic or therapeutic treatment regimen can be planned which is effective to treat the particular subject. The effective amount for any particular application can vary depending on such factors as the disease or condition being treated, the size of the subject and the severity of the disease or condition. One of ordinary skill in the art can empirically determine the effective amount of materials associated with the disclosure without necessitating undue experimentation.

The present disclosure also relates to risk-stratifying subjects based on their genetic profile. Through the use of technologies such as gene microarrays, based upon a gene expression profile, children and adults with septic shock can now be risk stratified by predicted mortality with great accuracy. Examination of the up-regulated genes that drive mortality in sepsis reveals that many of them are inflammatory proteins. Therefore, technology described herein can be used to tailor the bioactive circuit to individual patients to remove inflammatory mediators based on the genetic fingerprint from microarray technology.

The present disclosure also relates to the use of methods and compositions described herein for detecting or analyzing the presence of a substance in a biological fluid, such as for diagnostic and/or prognostic purposes. In some options, biological fluid, such as blood, is removed from a subject and is contacted with a polymer or particle attached to an agent such as an antibody, and is analyzed during or after contact with the polymer or particle. In some options, biological fluid, such as blood, is analyzed during or after circulation through an extracorporeal circuit. For example, in some cases, blood from a subject is circulated through an extracorporeal circuit that contains a chamber, wherein at least one surface of the chamber is coated with a specific agent such as an antibody, allowing for the presence and/or quantity of a specific substance such as a protein in the biological fluid to be analyzed.

Magnetically-susceptible particles associated with the disclosure, when it is desired to administer them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

Non-limiting examples of inflammatory disorders that may be treated as discussed herein include: sepsis or septic shock, acute or adult respiratory distress syndrome (ARDS), acute lung injury (ALI), Acne vulgaris, Asthma, Autoimmune diseases, Celiac disease, Chronic prostatitis, Glomerulonephritis, Hypersensitivities, Inflammatory bowel diseases, Pelvic inflammatory disease, Reperfusion injury, Rheumatoid arthritis, Sarcoidosis, Transplant rejection, Vasculitis, Interstitial cystitis, Atherosclerosis, Allergies, Inflammatory myopathies such as dermatomyositis, polymyositis, and inclusion body myositis, Chediak-Higashi syndrome and chronic granulomatous disease.

Non-limiting examples of autoimmune diseases that may be treated as discussed herein include: systemic lupus erythematosus (SLE), rheumatoid arthritis (RA), scleroderma, Sjogren's syndrome, multiple sclerosis, insulin dependent diabetes mellitus, ulcerative colitis, Acute disseminated encephalomyelitis (ADEM), Addison's Disease, Agammaglobulinemia, Alopecia areata, Amyotrophic Lateral Sclerosis, Ankylosing Spondylitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome, Autoimmune progesterone dermatitis, Autoimmune thrombocytopenic purpura, Autoimmune urticarial, Autoimmune uveitis, Balo disease/Balo concentric sclerosis, Behçet's disease, Berger's disease, Bickerstaffs encephalitis, Blau syndrome, Bullous pemphigoid, Cancer, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, CREST syndrome, Crohns Disease, Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic gastroenteritis, Epidermolysis bullosa acquisita, Erythema nodosum, Erthroblastosis fetalis, Essential mixed cryoglobulinemia, Evan's syndrome, Fibrodysplasia ossificans progressive, Fibrosing aveolitis (Idiopathic pulmonary fibrosis), Gastritis, Gastrointestinal pemphigoid, Giant cell arteritis, Glomerulonephritis, Goodpasture's syndrome, Graves' disease, Guillain-Barré syndrome (GBS), Hashimoto's encephalopathy, Hashimoto's thyroiditis, Henoch-Schonlein purpura, Herpes gestationis (Gestational Pemphigoid), Hidradenitis suppurativa, Hypogammaglobulinemia, Idiopathic Inflammatory Demyelinating Diseases, Idiopathic pulmonary fibrosis, Idiopathic thrombocytopenic purpura (Autoimmune thrombocytopenic purpura), IgA nephropathy, Inclusion body myositis, Chronic inflammatory demyelinating polyneuropathy, Interstitial cystitis, Juvenile idiopathic arthritis (Juvenile rheumatoid arthritis), Kawasaki's Disease, Lambert-Eaton myasthenic syndrome, Leukocytoclastic vasculitis, Lichen planus, Lichen sclerosus, Linear IgA disease (LAD), Lou Gehrig's disease (Also Amyotrophic lateral sclerosis), Lupoid hepatitis (Autoimmune hepatitis), Lupus erythematosus, Majeed syndrome, Ménière's disease, Microscopic polyangiitis, Miller-Fisher syndrome (Guillain-Barre Syndrome), Mixed Connective Tissue Disease, Morphea, Mucha-Habermann disease (Pityriasis lichenoides et varioliformis acuta), Multiple sclerosis, Myasthenia gravis, Myositis, Narcolepsy, Neuromyelitis optica (Devic's Disease), Neuromyotonia, Occular cicatricial pemphigoid, Opsoclonus myoclonus syndrome, Ord's thyroiditis, Palindromic rheumatism, PANDAS (pediatric autoimmune neuropsychiatric disorders associated with streptococcus), Paraneoplastic cerebellar degeneration, Paroxysmal nocturnal hemoglobinuria (PNH), Parry Romberg syndrome, Parsonnage-Turner syndrome, Pars planitis, Pemphigus vulgaris, Pernicious anaemia, Perivenous encephalomyelitis, POEMS syndrome, Polyarteritis nodosa, Polymyalgia rheumatic, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatic fever, Sarcoidosis, Schizophrenia, Schmidt syndrome, Schnitzler syndrome, Scleritis, Scleroderma, Serum Sickness, Sjogren's syndrome, Spondyloarthropathy, Still's disease (Juvenile Rheumatoid Arthritis), Stiff person syndrome, Subacute bacterial endocarditis (SBE), Susac's syndrome, Sweet's syndrome, Sydenham chorea (PANDAS), Sympathetic ophthalmia, Systemic lupus erythematosis (Lupus erythematosis), Takayasu's arteritis, Temporal arteritis (also known as "giant cell arteritis"), Thrombocytopenia, Tolosa-Hunt syndrome, Transverse myelitis, Ulcerative colitis (one type of idiopathic inflammatory bowel disease "IBD"), Undifferentiated connective tissue disease different from Mixed connective tissue disease, Undifferentiated spondyloarthropathy, Urticarial vasculitis, Vasculitis, Vitiligo and Wegener's granulomatosis.

### Microfluidic Channels

As mentioned, certain options may use microfluidic channels, e.g., as a surface that is at least partially coated with a suitable agent such as an antibody. Microfluidic channels generally have widths or diameters of less than about 1 mm, and less than about 100 micrometers in some cases. In some options, larger channels may be used instead of, or in conjunction with, microfluidic channels for any of the options discussed herein. For examples, channels having widths or diameters of less than about 10 mm, less than about 9 mm, less than about 8 mm, less than about 7 mm, less than about 6 mm, less than about 5 mm, less than about 4 mm, less than about 3 mm, or less than about 2 mm may be used in certain instances. In all options, specified widths can be a smallest width (i.e. a width as specified where, at that location, the article can have a larger width in a different dimension), or a largest width (i.e. where, at that location, the article has a width that is no wider than as specified, but can have a length that is greater). Thus, for instance, the microfluidic channel may have an average cross-sectional dimension (e.g., perpendicular to the direction of flow of fluid in the microfluidic channel) of less than about 1 mm, less than about 500 microns, less than about 300 microns, or less than about 100 microns. In some cases, the microfluidic channel may have an average diameter of less than about 60 microns, less than about 50 microns, less than about 40 microns, less than about 30 microns, less than about 25 microns, less than about 10 microns, less than about 5 microns, less than about 3 microns, or less than about 1 micron.

A channel may have any aspect ratio, e.g., an aspect ratio (length to average cross-sectional dimension) of at least about 1:1, at least about 2:1, more typically at least about 3:1, at least about 5:1, at least about 10:1, etc. As used herein, a "cross-sectional dimension," in reference to a fluidic or microfluidic channel, is measured in a direction generally perpendicular to fluid flow within the channel. A channel generally will include characteristics that facilitate control over fluid transport, e.g., structural characteristics and/or physical or chemical characteristics (hydrophobicity vs. hydrophilicity) and/or other characteristics that can exert a force (e.g., a containing force) on a fluid. The fluid within the channel may partially or completely fill the channel. In some cases the fluid may be held or confined within the channel or a portion of the channel in some fashion, for example, using surface tension (e.g., such that the fluid is held within the channel within a meniscus, such as a concave or convex meniscus). In an article or substrate, some (or all) of the channels may be of a particular size or less, for example, having a largest dimension perpendicular to fluid flow of less than about 5 mm, less than about 2 mm, less than about 1 mm, less than about 500 microns, less than about 200 microns, less than about 100 microns, less than about 60 microns, less than about 50 microns, less than about 40 microns, less than about 30 microns, less than about 25 microns, less than about 10 microns, less than about 3 microns, less than about 1 micron, less than about 300 nm, less than about 100 nm, less than about 30 nm, or less than about 10 nm or less in some cases. In one option, the channel is a capillary. In some cases, the device may contain one or more chambers or reservoirs for holding fluid. In some cases, the chambers may be in fluidic communication with one or more fluid transporters and/or one or more microfluidic channels.

A variety of materials and methods, according to the present disclosure, can be used to form the device, e.g., microfluidic channels. For example, various components of the disclosure can be formed from solid materials, in which the channels can be formed via micromachining, film deposition processes such as spin coating and chemical vapor deposition, laser fabrication, photolithographic techniques, etching methods including wet chemical or plasma processes, and the like. See, for example, Scientific American, 248:44-55, 1983 (Angell, *et al*).

In one set of options, various components of the systems and devices disclosed herein can be formed of a polymer, for example, an elastomeric polymer such as polydimethylsiloxane ("PDMS"), polytetrafluoroethylene ("PTFE" or Teflon®), or the like. For instance, according to one option, a microfluidic channel may be implemented by fabricating the fluidic system separately using PDMS or other soft lithography techniques (details of soft lithography techniques suitable for this option are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373).

Other examples of potentially suitable polymers include, but are not limited to, polyethylene terephthalate (PET), polyacrylate, polymethacrylate, polycarbonate, polystyrene, polyethylene, polypropylene, polyvinylchloride, polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The device may also be formed from composite materials, for example, a composite of a polymer and a semiconductor material.

In some options, various components of the disclosure are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one option, the hardenable fluid comprises a polymeric liquid or a liquid polymeric precursor (i.e. a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for options where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the disclosure include those formed from precursors including the chlorosilanes such as methylchlorosilanes, ethylchlorosilanes, phenylchlorosilanes, etc.

Silicone polymers are used in certain options, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of the microfluidic structures disclosed herein. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, about an hour. Also, silicone polymers, such as PDMS, can be elastomeric and thus may be useful for forming very small features with relatively high aspect ratios, necessary in certain options disclosed herein. Flexible (e.g., elastomeric) molds or masters can be advantageous in this regard.

One advantage of forming structures such as microfluidic structures of the disclosure from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, components can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the presentdisclosure, as well as overall molding techniques, are described in the art, for example, in an article entitled "Rapid Prototyping of Microfluidic Systems and Polydimethylsiloxane," Anal. Chem., 70:474-480, 1998 (Duffy *et al*.).

### Kits

Also disclosed herein, the present disclosure relates to a kit including one or more of the compositions previously discussed. A "kit," as used herein, typically defines a package or an assembly including one or more of the compositions of the disclosure, and/or other compositions associated with the disclosure, for example, as previously described. Each of the compositions of the kit, if present, may be provided in liquid form (e.g., in solution), or in solid form (e.g., a dried powder). In certain cases, some of the compositions may be constitutable or otherwise processable (e.g., to an active form), for example, by the addition of a suitable solvent or other species, which may or may not be provided with the kit. Examples of other compositions that may be associated with the disclosure include, but are not limited to, solvents, surfactants, diluents, salts, buffers, emulsifiers, chelating agents, fillers, antioxidants, binding agents, bulking agents, preservatives, drying agents, antimicrobials, needles, syringes, packaging materials, tubes, bottles, flasks, beakers, dishes, frits, filters, rings, clamps, wraps, patches, containers, tapes, adhesives, and the like, for example, for using, administering, modifying, assembling, storing, packaging, preparing, mixing, diluting, and/or preserving the compositions components for a particular use, for example, to a sample and/or a subject.

A kit as disclosed herein may, in some cases, include instructions in any form that are provided in connection with the compositions of the disclosure in such a manner that one of ordinary skill in the art would recognize that the instructions are to be associated with the compositions of the disclosure. For instance, the instructions may include instructions for the use, modification, mixing, diluting, preserving, administering, assembly, storage, packaging, and/or preparation of the compositions and/or other compositions associated with the kit. In some cases, the instructions may also include instructions for the use of the compositions, for example, for a particular use, e.g., to a sample. The instructions may be provided in any form recognizable by one of ordinary skill in the art as a suitable vehicle for containing such instructions, for example, written or published, verbal, audible (e.g., telephonic), digital, optical, visual (e.g., videotape, DVD, etc.) or electronic communications (including Internet or web-based communications), provided in any manner.

In some options, the present disclosure is directed to methods of promoting one or more options of the disclosure as discussed herein. As used herein, "promoted" includes all methods of doing business including, but not limited to, methods of selling, advertising, assigning, licensing, contracting, instructing, educating, researching, importing, exporting, negotiating, financing, loaning, trading, vending, reselling, distributing, repairing, replacing, insuring, suing, patenting, or the like that are associated with the systems, devices, apparatuses, articles, methods, compositions, kits, etc. of the disclosure as discussed herein. Methods of promotion can be performed by any party including, but not limited to, personal parties, businesses (public or private), partnerships, corporations, trusts, contractual or sub-contractual agencies, educational institutions such as colleges and universities, research institutions, hospitals or other clinical institutions, governmental agencies, etc. Promotional activities may include communications of any form (e.g., written, oral, and/or electronic communications, such as, but not limited to, e-mail, telephonic, Internet, Web-based, etc.) that are clearly associated with the disclosure.

In one set of options, the method of promotion may involve one or more instructions. As used herein, "instructions" can define a component of instructional utility (e.g., directions, guides, warnings, labels, notes, FAQs or "frequently asked questions," etc.), and typically involve written instructions on or associated with the disclosure and/or with the packaging of the disclosure. Instructions can also include instructional communications in any form (e.g., oral, electronic, audible, digital, optical, visual, etc.), provided in any manner such that a user will clearly recognize that the instructions are to be associated with the disclosure, e.g., as discussed herein.

Technologies described herein offer significant advantages over previously-developed technologies. For example, in some cases the advantage over existing extracorporeal systems (such as plasmapheresis or hemofiltration) of conferring great specificity through surface modification; the advantage over existing monoclonal antibody techniques of providing a time-limited effect; and the ability to remove multiple proteins at a time (depending on how many monoclonal antibodies are incorporated into the system) while preserving specificity.

The following examples are intended to illustrate certain options of the present disclosure, but do not exemplify the full scope of the invention.

### EXAMPLES

### Example 1: Construction of an antibody-functionalized extracorporeal circuit capable of reducing circulating levels of a specific substance, such as a cytokine, in biological fluids

Sepsis can be caused by severe infection and can lead to induction of either inflammatory or anti-inflammatory cytokines depending on numerous factors. Sepsis is thought to involve at least two stages: an initial inflammatory burst responsible for hypotension and organ dysfunction, followed by an anti-inflammatory response resulting in immune suppression. Using methods and devices described herein, the sepsis syndrome is modulated by targeted cytokine removal that is temporally customized to the stage of the disease. Numerous trials have been conducted in the past with agents intended to modulate the immune system in sepsis. Although early survival was improved, long term survival was no different (and perhaps even worse). Cytokine removal by pheresis, filtration and hemodialysis effectively attenuates cytokine levels (non-specifically, removing both types of cytokines), but does not improve clinical outcomes. Here, a biologically active extracorporeal circuit is developed, capable of highly specific cytokine removal that is temporally limited to the appropriate phase of the sepsis syndrome.

Using methods and devices described herein, the sepsis syndrome can be attenuated at any point throughout its progression by manipulating candidate cytokines at discrete time points. Biologically active extracorporeal circuits are described that are capable of modifying the composition of circulating biologic fluids with great specificity. Initially, interleukin-6 (IL-6) is targeted as an example of a specific cytokine. Whether by functional microgels affixed to a surface, or by circulating functional ferromagnetic nanoparticles (FFN) that can be removed with a magnetic field, or by any other functional polymeric system, the result is to attenuate IL-6 levels in a septic subject. Attenuation of a cytokine, such as IL-6, with biologically active circuits described herein can modify the extent to which the heart and kidneys develop dysfunction in septic subjects.

### Use of microgels to design, produce and characterize a biologically active extracorporeal circuit (BAEC) capable of reducing circulating levels of a specific substance, such as IL-6, in biological fluids

Here, microgels are functionalized with monoclonal antibodies (MAb) to create functional microgels capable of cytokine removal from biologic fluids. An example of a test biologic fluid consists of fetal bovine serum deliberately enriched with IL-6 and a control cytokine TNF-alpha (TNF-α). Functional microgels are used to construct ferromagnetic nanoparticles (FFN) (microgel microparticles with a particulate iron core), or used for surface modification of experimental extracorporeal circuits (EC). With the first approach, FFN:IL-6 complexes are removed by application of an external magnetic field to the circuit. In the second system, modified extracorporeal circuitry immobilizes IL-6 to the luminal surface from circulating test biological fluid as it passes through the circuit. Removal efficiency, capacity and specificity are characterized by quantifying cytokine concentrations in the test fluid before and after treatment with each BAEC system.

As an example, a semi-interpenetrating polymer network is constructed with monoclonal antibodies (such as rat anti IL-6 antibodies) anchored to subunits throughout. The resulting microgel is used to develop two types of bioactive extracorporeal circuits (targeting circulating IL-6).

For the first BAEC, the semi-interpenetrating polymer network is modified to create nanoparticles with super paramagnetic cores. These ferromagnetic nanoparticles bind a specific protein such as IL-6 and form complexes to be removed from circulation when an external magnetic field is applied to a reservoir in the circuit. Encapsulation of magnetic particles inside organic swelling particles is accomplished according to methods known in the art. Successful binding of IL-6 and efficient magnetically driven removal from the circulation is used to modify complex biological fluids.

The second BAEC system is developed by coating a surface, such as the luminal surface, of an extracorporeal circuit with a semi-interpenetrating polymer network. Surface modification with microgel systems is accomplished according to methods known in the art and is used here for covalently tethering the microgel to the surface of the circuit.

Both systems are tested for specificity, removal efficiency and removal capacity to determine that they can remove IL-6 from complex biological solutions under conditions that approximate clinical reality.

A microgel system capable of immobilizing specific proteins from a mixture of biological molecules is described in PCT Application No. PCT/US2012/026008, filed on February 22, 2012. In some options, particles are produced that have a ligand chelating moiety distributed throughout the entire matrix. This system has a high ligand density, superior performance compared to prior technologies and is easily penetrated by proteins in solution.

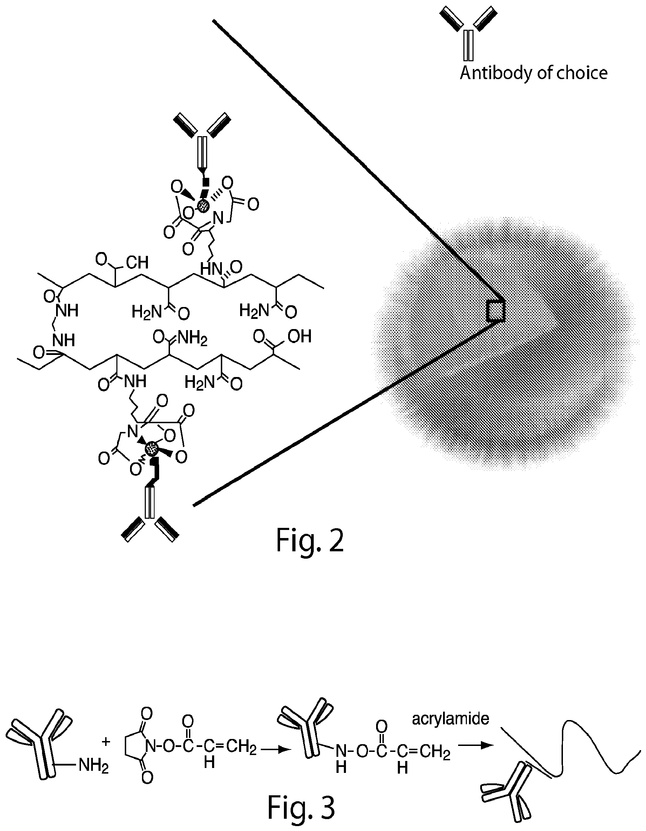

Polymers, such as polymers within microgel systems, can be functionalized with antibodies to targeted proteins and anchored to surfaces. Alternatively, super paramagnetic magnetite particles are incorporated into the core of the matrix to create magnetic scavenging particles that are injected and then collected by magnetizing the fluid in which they are suspended (figure 2).

### Methods

### Synthesis of the microgel:MAb

Synthesis of the antibody semi-interpenetrating polymer network hydrogel is accomplished by chemically modifying monoclonal rat anti-rat IL-6 IgG (RAIL-6) by coupling it with N-succinimidylacrylate (NSA) in phosphate buffer solution, using methods known in the art (Miyata et al. (1999) Nature 399(6738):766-9; Shoemaker et al. (1987) Applied Biochemistry and Biotechnology 15(1):11-24). NSA is added to a phosphate buffer solution (0.02 M, pH 7.4) containing RAIL-6 IgG (NSA/RAIL-6 IgG molar ratio is 6:1), and the reaction is incubated at 36°C for one hour to introduce the vinyl groups into the RAIL-6 IgG. The resultant vinyl(RAIL-6 IG) is purified with dialysis tubing. Acrylamide is added to the vinyl(RAIL-6 IG) solution, together with 0.1 M aqueous ammonium persulphate and 0.8 M aqueous N,N,N',N'-tetramethylethylenediamine (TEMED) as redox initiators, and the copolymerization is performed at 25°C for 3 hours to synthesize the polymerized RAIL-6 IgG (figure 3).

### BAEC Surface modification

Methods of functionalizing tubes with polymeric microgel films are derived from previous methods based on plasma-induced graft polymerization of poly acrylic acid (Singh et al. (2007) Biomacromolecules 8(10):3271-5. In order to make the method more general and to give the adherent microgel film more stability in biological environments, a photoaffinity label, viz., aminobenzophenone can be introduced onto the surface. Upon excitation with UV irradiation, molecules of the benzophenone family have the ability to abstract an aliphatic hydrogen atom from any nearby polymer chain forming a covalent carbon-carbon bond. When a microgel is present in the close vicinity of the benzophenone, the benzophenone can serve as a glue between the substrate and the microgel film.

### Ferromagnetic particles

Magnetic microgel nanoparticles are produced by embedding the super paramagnetic magnetite within the polymer using customary suspension, emulsion or precipitation polymerization during the microgel fabrication.

### Preparation of the biological test solution

Interleukin-6 is added to fetal bovine serum (FBS) in three concentrations: 20, 110 and 200 pg/ml. These values are derived from previous investigators measuring IL-6 concentrations in septic rats over time. As a control, TNF-alpha is added to the solution at 20 pg/ml.

### Experimental Setup

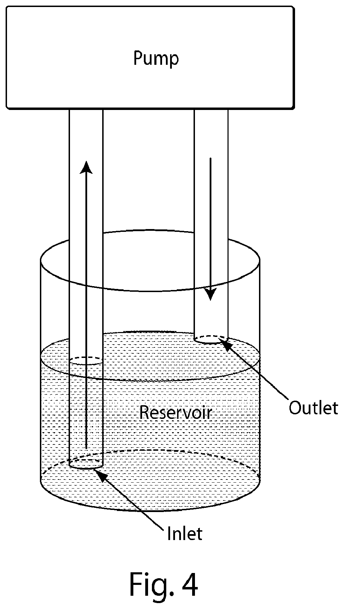

For both BAEC systems, circuits consist of a roller pump and a 30-cm polyvinyl chloride loop of tubing (internal diameter of 3 mm) containing a Medtronic R-14 silicone reservoir bladder and 3-way stopcock (figure 4). The circuit is filled with the biological test solution and the roller pump is operated to obtain a flow rate of 30-40 ml/min. These flow rates are analogous to 100 ml/kg/min for adult male Sprague-Dawley rats (350-450 grams), which are used in the animal studies. Depending on the investigation, the bladder surface is either modified with functional microgel to scavenge IL-6 or an external magnetic field is applied to remove circulating FFN:IL-6 complexes that form after FFN injection into the circuit. The biological solution undergoes treatment by one of the two methods.
1. Surface modified EC for IL-6 removal: The biologic suspension undergoes circulation through the circuit for predetermined durations in order to characterize the IL-6 removal efficiency and removal capacity. Specificity is evaluated using TNF-alpha (TNF-α) as a control. Initially these intervals are 1, 2 and 4 hours. Adjustments in duration and surface chemistry are made as needed to optimize performance before advancing to animal trials. The treated biologic test solution is removed from the 3-way stopcock for ELISA and cell culture experiments.
2. Magnetically assisted IL-6 removal: The microparticles are injected into one of the ports of the reservoir bladder and allowed to circulate for predetermined time intervals (initially 1, 2 and 4 hours). At the end of the circulation time, an external magnetic field is applied to the reservoir bladder to immobilize the functional ferromagnetic nanoparticle:IL-6 complexes from circulation. The treated biologic suspension is removed from the 3-way stopcock for ELISA and cell culture experiments. The amount of microparticles necessary to attenuate IL-6 levels is determined as their performance is characterized.

### Evaluation of cytokine concentrations

Concentrations of TNF-alpha (TNF-α) and IL-6 in the treated biological test solution are measured by enzyme-linked immunosorbent assay (ELISA) using rat polyclonal anti-rat cytokine antibodies.

### Example 2: Characterization of the in vitro biologic impact of treating IL-6 enriched biological fluids with biologically active extracorporeal circuits.

Here, cytokine enriched test fluid is circulated through one of the two BAEC systems. For the first treatment group, IL-6 targeted FFNs are injected into the circuit and allowed to circulate for predetermined time intervals followed by removal of FFN:IL-6 complexes by application of an external magnetic field. The second treatment group consists of an EC in which the luminal surface has been modified by the IL-6 targeted FM, resulting in IL-6 immobilization to the EC lumen. Rat cardiomyocytes are cultured in the biologic fluid before and after treatment with the BAEC and assessed for contractility and viability. These experiments demonstrate the influence of the BAEC on cell viability and contractility by IL-6 removal.

There is growing evidence that IL-6 possesses negative inotropic properties, based on experiments involving culturing cardiomyocytes in media containing cytokine enriched biological fluids. *In vitro* parameters of contractility as well as cytotoxicity should be influenced by IL-6 concentration. Cytokine enriched biological fluids treated with the BAEC systems described herein result in IL-6 depletion from the test fluid. The ability of each BAEC system to modify cardiomyocyte depression and cytotoxicity is demonstrable by comparing these parameters in cells cultured in biological fluids before and after IL-6 removal. These experiments provide insight into the biologic influence of these bioactive circuits. Furthermore, these experiments provide the framework for treatment of biologic fluid to remove the candidate protein(s) and characterization of the effect of protein removal on cellular function (cell type selection guided by hypothesized influence of protein on cellular function).

### Methods

Ventricular myocytes are obtained from adult male Sprague-Dawley rats (300-400 g) and prepared by the collagenase perfusion method using standard enzymatic techniques previously described (Lee et al. (1979) Nature 278(5701):269-71; Louch et al. (2011) Journal of Molecular and Cellular Cardiology 51(3):288-98). The isolated cells are diluted to a concentration of 10⁴ cells/mL and incubated in 24-well plates (2 mL aliquots) at 37°C, 5% CO₂ with biological test solution for periods of 0-48 hours.

### Detection of Myocardial Depressant Activity

The method used is adapted from that previously described by Pathan et al. (2002) Critical Care Medicine 30(10):2191-8. Ventricular myocytes in Tyrode's solution (NaCl 140 mM, KCl 6 mM, MgCl₂ 1 mM, CaCl₂ 2 mM, glucose 10 mM, N-hydroxy ethyl piperazine-N-2-ethane sulfonic acid 10 mM, with NaOH to adjust the pH to 7.3-7.4) are placed in a Perspex chamber on the stage of an inverted microscope and allowed to spontaneously attach to the surface. After 5 minutes, the cells are superfused with Tyrode's solution at a rate of 1 ml/min and warmed to 37°C. Contraction is induced by field stimulation (0.5 Hz, 30 V, 0.2 msecs). An individual cell is displayed on a monitor. An edge detection device is used to measure cell length, so that changes in length with contractions are transmitted as an electrical signal. Myocyte contractility is characterized by contraction amplitude, speed of contraction, and speed of relaxation.

### Determination of ventricular myocyte viability

To quantitatively assess cell viability after adding test solution, a colormetric assay (3-(4,5-dimethylthiazol-2-yl)-2,5-diphenyl tetrazolium bromide [MTT] kit, Promega G4100; Madison, WI) is used. In live cardiac myocytes, the yellow tetrazolium salt is metabolized into insoluble purple formazen crystals. A detergent is added to solubilize the crystals and the color is then quantified by spectrophotometric means.

### Effect of test substance on ventricular myocytes

Cell contractility is characterized immediately before and 5 minutes after exposure to test substance to assess acute changes in myocyte contraction. Latent effects are measured after 24 - 48 hours of incubation. Cell viability is quantified after 24 and 48 hours of incubation in test solution.

### Example 3: Immobilization of circulating cytokines from septic rats using the biologically active extracorporeal circuitry.

Interleukin-6 has been implicated in the development of myocardial depression in meningococcal sepsis, as well as acute kidney injury. Here, extracorporeal circuitry techniques are used in rats that have been rendered septic by the cecal ligation and puncture (CLP) model then connected to BAEC systems for IL-6 removal. Cytokine concentrations are measured at predetermined time intervals. Myocardial function is assessed by echocardiography and kidney injury is characterized by serum BUN and creatinine, as well as postmortem
histology. IL-6 levels are correlated with measurements of myocardial function and kidney injury.

Echocardiography is the foremost method for imaging the cardiovascular system in small animals because it is noninvasive, versatile, widely available and well suited for serial studies. Indices of global systolic function can be easily and reliably obtained. Elevations in blood urea nitrogen (BUN) and serum creatinine have been measured with IL-6 associated kidney injury in mice, and correlated with post-mortem histologic markers of injury. For these reasons, echocardiography is used to serially measure myocardial function, and BUN and creatinine to measure renal function as IL-6 is removed from the circulation.

In addition to possessing negatively inotropic properties, IL-6 has been demonstrated to mediate acute kidney injury (AKI). Therefore, rats rendered septic by cecal ligation and puncture should manifest myocardial depression and AKI that directly correlates with circulating IL-6 concentration. Here, the BAEC system is tested for attenuation of IL-6 levels in septic rats. IL-6 levels are measured over time while undergoing treatment with the bioactive circuits. Echocardiography is used to measure myocardial function; blood urea nitrogen, creatinine and postmortem histology are used to measure the degree of AKI. These parameters are correlated with circulating IL-6 levels, providing insight into how these systems perform *in vivo.*

### Methods

### Induction of septic shock and initiation of the veno-venous circuit

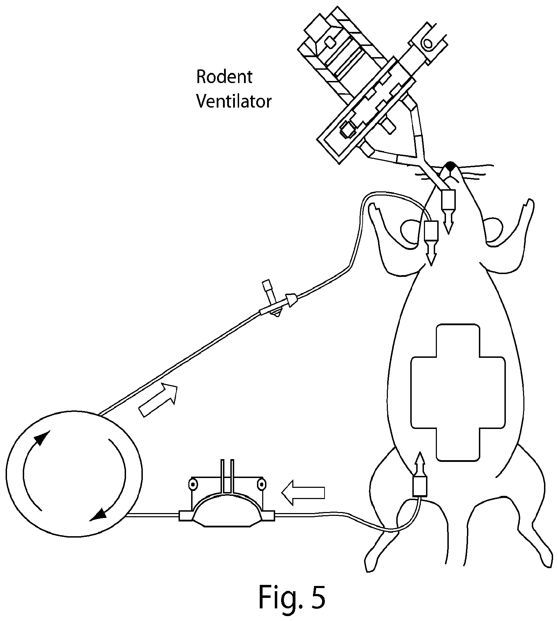

Here, septic rats are connected to a veno-venous circuit (figure 5). After induction of anesthesia with intraperitoneal sodium pentobarbital (50 mg/kg), adult male Sprague-Dawley rats are rendered septic by cecal ligation and puncture (CLP). A 50 mL/kg subcutaneous bolus of normal saline as fluid resuscitation is administered and the animals returned to their cages and allowed food and water ad libitum. Seven hours after CLP animals are reanesthesized and intubated with an angiocatheter via a tracheotomy. Mechanical ventilation is performed with a Harvard Rodent Ventilator at a tidal volume of 10 mL/kg to maintain an arterial PO₂ between 35 and 45 mm Hg. The right femoral vein and right jugular veins are isolated and cannulated with appropriately sized angiocatheters. Heparin (1000 IU) is administered prior to initiation of flow on the circuit. The circuit is set to flow at a rate of 30-40 ml/min (100 ml/kg/min). Animals undergo treatment with either a surface modified EC, or magnetically assisted IL-6 removal. At predetermined time intervals myocardial function is monitored by echocardiography (ejection fraction, shortening fraction). BUN and serum creatinine are sampled to identify acute kidney injury (AKI). Circulating IL-6 and TNF-alpha concentrations are monitored by rat specific ELISA throughout treatment.

### Surface modified EC for IL-6 removal

Seven hours after CLP, animals assigned to the surface modified EC group undergo mechanical ventilation and placement onto the circuit. Blood is drawn from the 3-way stopcock and echocardiography is performed at 8, 12, 16, and 20 hours after CLP.

### Magnetically assisted IL-6 removal

Seven hours after CLP, animals assigned to the functional ferromagnetic microparticle group undergo mechanical ventilation and placement onto the circuit. Ferromagnetic microparticles are injected intravenously during the cannulation procedure. An external magnetic field is applied to the venous reservoir at 8, 12 and 16 hours after CLP to remove
opsonized cytokines from the circulation. Blood is drawn and echocardiography is performed at 8, 12, 16 and 20 hours after CLP.

### Histologic evaluation of kidneys

Several different pathophysiological mechanisms for sepsis-induced AKI have been proposed, with systemic cytokine storm or local cytokine reactions being a major feature. Recently, IL-6 mediated inflammation has been demonstrated to enhance renal injury after ischemia. Kidneys are removed from the rats following euthanasia and undergo fixation in formaldehyde, followed by dehydration with ethanol. Tissue is imbedded in Paraplast and sectioned, followed by deparafinization with xylene and staining with hematoxylin and eosin. The samples are then be evaluated for polymorphonuclear (PMN) leukocyte influx into the renal tissues. The total number of infiltrating leukocytes in cortical interstitial spaces are assessed quantitatively by counting the number of PMNs in 20 high power fields.

### Malondialdehyde (MDA) Measurement

MDA levels in kidney samples are determined as an indicator of lipid peroxidation resulting from inflammation. Kidney tissue samples are homogenized in a 1.15% KC1 solution and the homogenate added to a reaction mixture of SDS, acetic acid, thiobarbituric acid
and distilled water. After boiling the mixture, MDA is quantified by spectrophotometric means (650 nm).

### Immunohistochemical Analysis of ICAM-1 and P-Selectin

Localization of ICAM-1 and P-selectin in Kidney sections is determined as previously described (Patel et al. (2005) Journal of Pharmacology and Experimental Therapeutics 312(3): p. 1170-8. Kidney sections are incubated overnight at 4°C with primary anti-ICAM-1 or anti-P-selectin antibody [1:500 (v/v) in PBS]. After blocking endogenous avidin and biotin, specific labeling of antigen-antibody complex is visualized using chromogen diaminobenzidine.

### Statistical Analysis

All analyses are performed using IBM SPSS Statistics 19 software. Unless otherwise stated, all outcomes are measured on a continuous scale. Comparisons of contraction amplitude, percentage change in contraction amplitude and cell viability between groups is assessed with Mann-Whitney tests. The Kruskal-Wallis test is used to assess the difference in interleukin 6, BUN and creatinine concentrations between groups. Comparison between clinical groups for shortening fraction and ejection fraction is also be done with Kruskal-Wallis tests. For histologic scoring, data is analyzed using one-way analysis of variance.

### Example 4: Construction of a biologically active extracorporeal circuit (BAEC) involving surface modification of polymer tubing that is capable of selectively removing circulating cytokines from biological fluids.

A BAEC was developed using poly(dimethylsiloxane) (PDMS) tubing with an internal diameter of 3 mm. Initially, VEGF was targeted as an example of a specific cytokine, and the inner surface of the PDMS tubing was functionalized such that the polymeric substrate was connected via APTMS-NHS PEG maleimide linkers to anti-VEGF antibodies. This created a surface capable of selectively removing VEGF from biological fluids passing through the tube.

### Methods

### BAEC surface functionalization

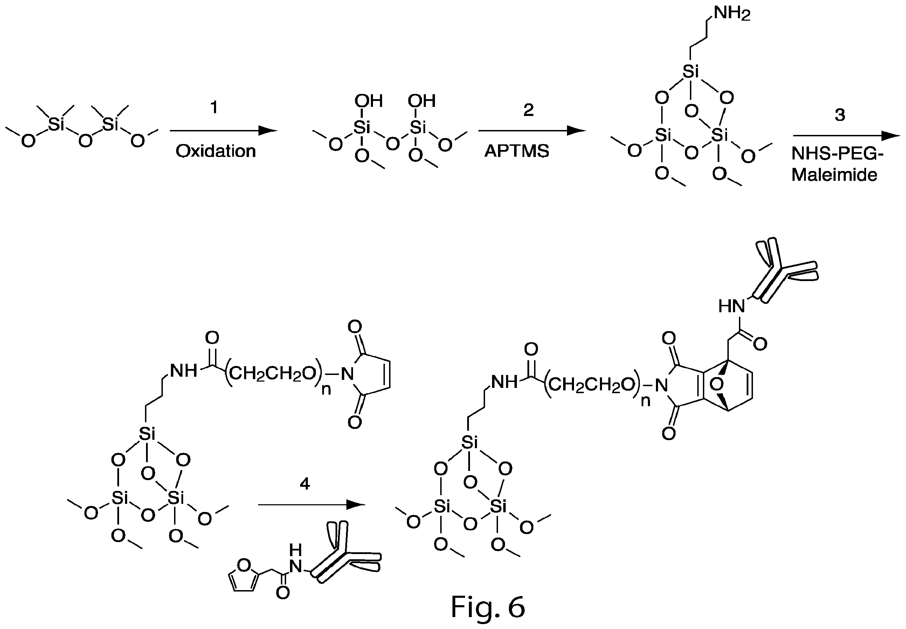

Functionalization of the luminal surface of the PDMS tubing was accomplished through four sequential reactions (figure 6). First, the PDMS surface was oxidized to create reactive Si-OH moieties using a plasma oxidizer for two minutes. Second, the activated PDMS surface was incubated with 5% (w/v) aminopropyltrimethoxysilane (APTMS) in dry acetone for 60 minutes to covalently graft APTMS to the silanol moieties. Third, NHS-PEG-maleimide was added at pH 8.5, resulting in an (APTMS)-(maleimide PEG) anchor attached to the PDMS surface. To prevent hydrolysis, the tubes were stored in 2-(N-morpholino)ethanesulfonic acid (MES) at pH 5.5. Fourth, an anti-VEGF antibody was added, and the (APTMS)-(maleimide PEG) anchor was covalently bound to thiol groups on the antibody. To reduce non-specific binding, the surface was incubated with 5% (w/v) bovine serum albumin (BSA) in phosphate buffered saline (pH 7.4) for 48 hours.

### Preparation of the biological test solution

Suspensions of VEGF (targeted cytokine) and IL-6 (untargeted control cytokine) were prepared at a concentration of 2000 pg/ml in a solution of 5% (w/v) BSA in phosphate buffered saline (pH 7.4) at 37°C.

### Experimental Setup

The biological test solution was placed into a reservoir with an outlet and an inlet connected by PDMS tubing functionalized with anti-VEGF antibody. Continuous circulation of the reservoir fluid through the circuit was driven by a peristaltic pump at a physiologically relevant flow rate of 40 mL/min at 37°C.

### Evaluation of cytokine concentrations

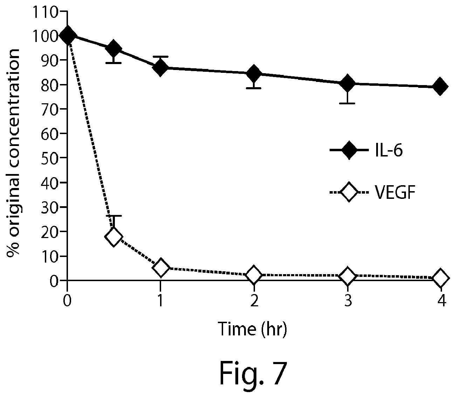

Concentrations of VEGF and IL-6 in the treated biological test solution were quantified over time by an enzyme-linked immunosorbent assay (ELISA). All circulating VEGF was removed from the solution after 4 hours, whereas IL-6 was largely unaffected (figure 7).

### Example 5: Construction of a biologically active extracorporeal circuit (BAEC) involving surface modification of microfluidic channels that is capable of selectively removing circulating cytokines from biological fluids.

BAECs will be developed using microfluidic channels. PDMS microfluidic channels have been constructed with internal diameters ranging from 20 to 80 micrometers (µm). Using the methods of Example 4, the inner surface of the PDMS microfluidic channels will be functionalized such that the polymeric substrate is connected by maleimide linkers to anti-VEGF antibodies. This will create a system capable of selectively removing VEGF from biological fluids passing through the microfluidic channels.

### Example 6: Demonstration of biologic effects of cytokine scavenging in a cell culture model.

VEGF and IL-6 enhance microvascular permeability (i.e. capillary leak) in vivo and increase endothelial cell monolayer permeability in vitro. Endothelial cell monolayers grown on semi-permeable membranes are impermeable to albumin. When incubated in VEGF and/or IL-6, they become permeable over time (analogous to developing capillary leak). This can be quantified by the diffusive albumin permeability assay. Human umbilical vein endothelial cells (HUVEC) are incubated with media containing VEGF and/or IL-6 for predetermined time intervals (untreated cytokine media). Separate HUVEC monolayers are be incubated in cytokine-enriched media that has undergone circulation through functionalized circuitry for various durations of time to remove VEGF and/or IL-6 (i.e. treated media). Diffusive albumin permeability is quantified in each population to document the influence of selective cytokine filtration on the development of capillary leak.

## Claims

1. A device, comprising:
an extracorporeal circuit comprising a tube having an inner surface and an antibody or antigen-binding fragment thereof bound to a substrate forming the inner surface of the tube, wherein the substrate comprises a polymeric microgel.

2. The device of claim 1, wherein the antibody or antigen-binding fragment thereof is specific to an inflammatory cytokine.

3. The device of claim 2, wherein the antibody or antigen-binding fragment thereof is specific to IL-6 or VEGF.

4. The device of any of claims 1-3, wherein the antibody or antigen-binding fragment thereof is attached to the polymeric microgel, optionally wherein the antibody or antigen-binding fragment thereof is attached to the polymeric microgel by a crosslinker, optionally wherein the crosslinker comprises aminopropyltrimethoxysilane (APTMS) or NHS-PEG-maleimide.

5. The device of claim 4, wherein the tube comprises silicone and/or poly(dimethylsiloxane).

## Patentansprüche

1. Vorrichtung, Folgendes umfassend:
einen extrakorporalen Kreislauf, der einen Schlauch mit einer Innenoberfläche und einen Antikörper oder ein antigenbindendes Fragment davon umfasst, der/das an ein Substrat gebunden ist, das die Innenoberfläche des Schlauchs ausbildet, wobei das Substrat ein Polymer-Mikrogel umfasst.

2. Vorrichtung nach Anspruch 1, wobei der Antikörper oder das antigenbindende Fragment davon für ein inflammatorisches Cytokin spezifisch ist.

3. Vorrichtung nach Anspruch 2, wobei der Antikörper oder das antigenbindende Fragment davon für IL-6 oder VEGF spezifisch ist.

4. Vorrichtung nach einem der Ansprüche 1-3, wobei der Antikörper oder das antigenbindende Fragment davon mit dem Polymer-Mikrogel verbunden ist, optional wobei der Antikörper oder das antigenbindende Fragment davon mit dem Polymer-Mikrogel durch einen Vernetzer verbunden ist, optional wobei der Vernetzer Aminopropyltrimethoxysilan (APTMS) oder NHS-PEG-Maleimid umfasst.

5. Vorrichtung nach Anspruch 4, wobei der Schlauch Silicon und/oder Poly(dimethylsiloxan) umfasst.

## Revendications

1. Dispositif, comprenant :
un circuit extracorporel comprenant un tube présentant une surface intérieure et où un anticorps ou un fragment de liaison à l'antigène de celui-ci est lié à un substrat formant la surface intérieure du tube, le substrat comprenant un microgel polymère.

2. Dispositif selon la revendication 1, l'anticorps ou le fragment de liaison à l'antigène de celui-ci étant propre à une cytokine inflammatoire.

3. Dispositif selon la revendication 2, l'anticorps ou le fragment de liaison à l'antigène de celui-ci étant propre à l'IL-6 ou de VEGF.

4. Dispositif selon l'une quelconque des revendications 1 à 3, l'anticorps ou le fragment de liaison à l'antigène de celui-ci étant fixé au microgel polymère, l'anticorps ou le fragment de liaison à l'antigène de celui-ci étant facultativement fixé au microgel polymère par un agent de réticulation, l'agent de réticulation comprenant facultativement de l'aminopropyltriméthoxysilane (APTMS) ou du NHS-PEG-maléimide.

5. Dispositif selon la revendication 4, dans lequel le tube comprend du silicone et/ou du poly(diméthylsiloxane).
